(19) **Europäisches Patentamt**
**European Patent Office**
**Office européen des brevets**

(11) **EP 4 778 571 A1**

(12) **EUROPEAN PATENT APPLICATION**

(43) Date of publication:
22.07.2026 Bulletin 2026/30

(21) Application number: 25315017.1

(22) Date of filing: 21.01.2025

(51) International Patent Classification (IPC):
**A61N 7/02** (2006.01)  **A61B 8/00** (2006.01)
**A61B 90/00** (2016.01)  **A61N 7/00** (2006.01)

(52) Cooperative Patent Classification (CPC):
**A61N 7/02; A61B 8/00;** A61B 90/98;
A61B 2090/378; A61N 2007/0073

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Theraclion**
**92240 Malakoff (FR)**

(72) Inventors:
• ANQUEZ, Jérémie
75013 Paris (FR)

• GRISET, Anthony
78210 Saint-Cyr-L'Ecole (FR)
• GEROLD, Björn
78000 Versailles (FR)
• LOUVEL, Eric
75013 Paris (FR)
• FLODERER, Jean-Baptiste
67000 Strasbourg (FR)

(74) Representative: **Hepp Wenger Ryffel AG**
**Friedtalweg 5**
**9500 Wil (CH)**

(54) **A HITU DEVICE**

(57) The invention discloses a HITU device (400) comprising a radiofrequency generator (404), a first controller (408), and a user interface (407). The radiofrequency generator (404) is configured for delivering a high-power radiofrequency signal within at least a first frequency band and a second frequency band. The first controller (408) is configured for switching between the first and the second frequency band. The user interface (407) comprises a switch which, when activated, causes the first controller (408) to change the output frequency of the radiofrequency generator (404) from the first to the second frequency band or from the second to the first frequency band.

Fig. 1

## Description

The present invention relates to a HITU device and associated methods according to the preamble of the independent claims.

Ultrasonic waves are known in the prior art as a means for therapy and imaging. Lower ultrasonic frequencies provide a better penetration into tissues while higher ultrasonic frequencies enable a better focusing.

In known applications, e.g. in imaging and High-Intensity Therapeutic Ultrasound (HITU) such as High Intensity Focused Ultrasound (HIFU), a frequency is often selected which forms a compromise appropriate for a given application, e.g. to sacrificing, to an acceptable level, penetration to retain a certain focus and vice versa.

WO 02/32506 A1 discloses an imaging compatible device which employs ultrasound energy for thermal therapy. Directionality of the thermal therapy is effected through transducer structure and rotational control of the device. The depth of thermal therapy is controlled through simultaneous modulation of the device's operating frequencies and ultrasonic power.

US 2006/0206105 A1 discloses an apparatus for delivering and controlling thermal therapy to a volume of diseased tissue by using thermal imaging and other inputs to determine an acoustic treatment regime employing interstitial ultrasound applicators to deliver a required therapeutic temperature or thermal dose to the affected region in a body or organ.

US20180036558A1 discloses an interstitial ultrasound thermal ablation applicator for conformal treatment of an inhomogeneous tumor lesion.

US 9,931,245 B2 discloses a device for treatment of an ocular pathology having transducers made either in piezocomposite material or in piezoceramic material.

US 5,558,092 discloses methods and apparatus for performing diagnostic ultrasound simultaneously with the application of therapeutic ultrasonic waves.

The devices known in the prior art present several problems.

For one, to provide suitable frequencies, broadband radiofrequency amplifiers may need to be employed, which tend to be bulky and costly.

Thus, it is the aim of the current invention to solve these limitations by providing a versatile and robust HITU device.

This and other objects are achieved by the HITU device and the methods according to the characterizing portion of the independent claims of the invention.

The invention is directed to a HITU device comprising a radiofrequency generator, a first controller, and a user interface. The radiofrequency (RF) generator is configured for delivering a high-power radiofrequency signal within at least a first frequency band and a second frequency band. The first controller is configured for switching between the first and the second frequency band. The user interface comprises a switch which, when activated, causes the first controller to change the output frequency of the radiofrequency generator from the first to the second frequency band or from the second to the first frequency band. The first controller may be adapted to automatically switch the output frequency of the radiofrequency generator from the first to the second frequency band or from the second to the first frequency band. Additionally or alternatively, the switch may include a software which is adapted to automatically change between the first and second frequency bands without user input. Preferably, the first controller is further adapted to display an indication that a change occurred on the user interface

The RF generator may generate an electric signal which may be translated into a vibration signal (i.e. a sound wave) by a piezoelectric transducer.

The HITU device may comprise a memory for saving parameters.

The HITU device may comprise a treatment head. The treatment head my comprise an ultrasonic transducer.

Piezoceramic transducers may resonate at their fundamental frequency $f0$ but may also be driven at a frequency $3f0$ which is substantially three times the fundamental, often called the third harmonics. Other transducers type, such as piezocomposites are also known in the art and may be more broadband and may be used in a certain range of frequencies with a sufficient efficacy.

The ultrasound transducer may be a piezoceramics transducer and may have at least two frequency bands which encompass the fundamental frequency $f0$ and the third harmonics $3f0$ of the transducer. The skilled person understands that a certain variability resulting from the manufacturing of the transducers may be present is the mentioned frequencies.

The RF generator may comprise or consist of several interconnected devices (e.g. one for generating a signal, and one for amplifying and filtering) within the HITU device.

The HITU device may further comprise at least a first impedance matching circuit and a second impedance matching circuit. The first and second impedance matching circuits may be assigned to the first and the second frequency bands, respectively. A second controller configured to switch between the first and the second impedance matching circuits may be included in the HITU device. Additionally or alternatively, the first controller may be adapted to switch between the impedance matching circuits as well. The switch of the user interface, when activated, may cause the second controller (or first controller, as the case may be) to switch from the first to the second impedance matching circuit or from the second to the first impedance matching circuit. Preferably, the switching by the second controller is performed in synchronization with the first controller such that the impedance matching circuit which is selected by the second controller is assigned to a currently selected output frequency of the RF generator.

Thus, when the switch of the user interface is activated, the first controller may change the output frequency of the RF generator and the second controller may change the impedance matching for the impedance matching circuit corresponding to the output frequency band of the RF generator. Preferably, the impendance matching circuit comprises a first and a second circuit, wherein changing the impedance matching includes changing between the first and second circuit.

In a preferred embodiment, the treatment head comprises an ultrasonic transducer for therapy and/or an imaging means (e.g. an ultrasound imaging transducer). At least two impedance matching circuits may be connected to the therapy transducer and a second controller able to switch between the at least two impedance matching circuits may be present, e.g., in the treatment head. When the switch of the user interface is activated, the first controller may then change the output frequency of the RF generator and the second controller may change the impedance matching for the impedance matching circuit corresponding to the appro-priate output frequency band.

Switching the generator's output frequency and the impedance matching may be done simultaneously or consecutively. Both switches are typically made to consistent positions (to the same frequency band) before pulse delivery. Preferably, an error mechanism is implemented to forbid or interrupt the delivery of the pulse the frequencies of the RF generator and of the impedance matching circuit are not in consistent positions (which can be directly or indirectly assessed, e.g. based on reflected power measurement).

Preferably, the treatment head houses the first and/or the second impedance matching circuit. The first and second impedance matching circuits may be configured to match/correspond to the frequency bands that the RF generator may output.

Preferably, the at least two impedance matching circuits enable the transducer to substantially match a 50 Ohms impedance when driven at two different frequencies, preferably substantially corresponding to f0 and 3f0 for a piezoceramics transducer.

It may be advantageous to embed these impedance matching circuits within the treatment head and thus connecting them to the HITU transducer. Thereby, different treatment heads may be connected on the same HITU device since each treatment head carries its own impedance matching circuits. The impedance matching may have to be tuned for each individual transducer. Preferably, there is one impedance matching circuit for each different frequency at which the transducer may be driven by the RF generator.

Additionally or alternatively, a broadband impedance matching or no impedance matching may be used if the RF generator is configured to handle a high level of reverse signal and/or can deliver more power or the transducer (e.g. a piezo-ceramic transducer).

The HITU device may further comprise a frequency filter each for the first frequency bands and/or second frequency bands (e.g. a first frequency filter for the first frequency band and/or a second frequency filter for the second frequency band). The frequency filter may be configured to filter out at least the second and/or third harmonics of the corresponding frequency band.

Preferably, the frequency filter is embedded in the RF generator.

The HITU device may, in particular, comprise at least one filter for each of the at least two frequency bands, preferably such that the filter corresponding to each frequency band filters out at least the frequencies greater or equal to three times the signal frequency, most preferably greater or equal to twice the signal frequency.

Thereby, frequencies that should not be delivered to the patient may be avoided as they do not reach the ultrasound transducer. This provides additional safety, as certain frequencies may accidentally be transmitted/emitted by the transducer.

Such frequencies (which may be higher than the desired frequency) may be more absorbed by the tissues and could possibly induce skin burns, or limit the maximum power which can be emitted by the HITU device without inducing skin burns (e.g. if the maximum power thresholds were experimentally measured without filtering these frequencies).

Additionally or alternatively, a band-pass filter may be used which encompasses the first and second frequency bands.

In a preferred embodiment, the user interface is adapted to display a medical image, preferably an ultrasound image, with a representation of at least one of an affected region of a tissue, safety zones, and/or characteristic points of the HITU field based on a selected bandwidth or the selected frequency band.

For example, a reticle may be displayed on the live image representing a zone in which the target should be located for a therapeutic effect.

Different ultrasonic frequencies may lead to different physical phenomena. For example, a frequency of 1 MHz with a sufficient power may induce cavitation, while a higher frequency (e.g. 3 MHz) may mainly result in a thermal effect, leading to different areas of effect of the pulses depending on the pulse parameters such as the frequency. It is thus advantageous to adapt the display to adapt the positioning of the treatment head with respect to the target accordingly.

The output power of the RF generator may be above 200 W, preferably above 500 W, most preferably above 800 W.

The RF generator may comprise an automatic gain control (AGC) circuit and/or another way (e.g. a software correction) for the output power to be within 20%, preferably within 5%, of a setpoint.

The AGC (or any other power control) may adapted to be reset manually or automatically.

In one embodiment, the HITU device may send a forward power target to the RF generator. Based on this setpoint, the RF generator configures the electric command (e.g. an attenuator or a gain) to an appropriate value and enables the power output. The RF signal is amplified and sent to the HITU treatment head. During the pulse the forward power is constantly read via a dual directional coupler and controlled in closed loop by the electronic circuitry of the AGC. It compares the forward power target and the forward power read and constantly adjusts the electrical command.

In a preferred embodiment, the RF generator may also comprise a fixed software correction in the firmware of the microcontroller unit (MCU). The RF generator's calibration may be tuned for a fixed frequency and it may be possible to generate the RF signal within 10 % of the calibration frequency, which induces variations of the amplification value. The software correction permits to correct the forward power requested and the power measured based on a linear correction.

The HITU device may further have a feedback control mechanism configured to adapt a pulse characteristic based on a pulse history.

The pulse history may include data of at least one previously delivered pulse, for example characteristics such as frequency and/or power.

For example, the feedback control mechanism may keep in the memory a history of a ratio between a requested and a delivered power of one or several pulses (e.g. by storing in the memory the requested and the delivered power of the at least one last pulse and/or the ratio between the requested and the delivered power), thereby allowing to adapt the requested power to the actual performance of the generator. Advantageously, it may then be possible to use a setpoint which takes into account the actual performance of the RF generator as it may occur that an AGC fails to deliver a power which corresponds to the requested power, e.g. due to the HITU treatment head's possibly complex impedance.

It is also conceivable that the feedback control mechanism comprises several histories depending on the pulse characteristics and/or environmental conditions or one history and selects the relevant pulses within depending on the current pulse characteristics and/or environmental conditions.

In a preferred embodiment, the feedback control mechanism is implemented in a controller outside the RF generator. Alternatively, it is implemented in a separate controller within the RF generator and/or in the first and/or second controller mentioned above.

In a preferred embodiment, the feedback control mechanism is based on a history comprising N pulses (preferably with 0 < N < 20).

For example, the ratio between the requested and the delivered power at pulse k is stored as $\eta_k$ and the setpoint for a pulse k+1 is multiplied by a coefficient

$$\alpha_k = \frac{1}{N} \sum_{i=k-N}^{k-1} \eta_i \ .$$

In other embodiments, other formulas can be used (e.g. by taking the median instead of the average, and/or a weighted mean, and/or the maximum value). For example, if N = 1, and the power requested by the user for the last pulse was 100 W while the delivered power was 95 W, $\alpha_k = \frac{100}{95} \approx 1.05$ . Thus, if the user requests a power of 120 W, the actual power setpoint for the generator will be 120 × $\alpha_k \approx$ 126 *W.*

In a preferred embodiment, if N > 1, if the history comprises M pulses, with 0 < M < N, the coefficient $\alpha_k$ is still computed, e.g. as $\alpha_k = \frac{1}{M} \sum_{i=k-M}^{k-1} \eta_i$ . In an alternative embodiment, $\alpha_k$ is not computed until the history comprises at least L pulses, with 0 < L ≤ N.

Preferably, the feedback control mechanism is configured to reset the pulse history if a change in pulse characteristics or environmental characteristics is above a threshold.

For example, the feedback control mechanism's history may be reset when the pulse characteristics or environmental characteristics change substantially.

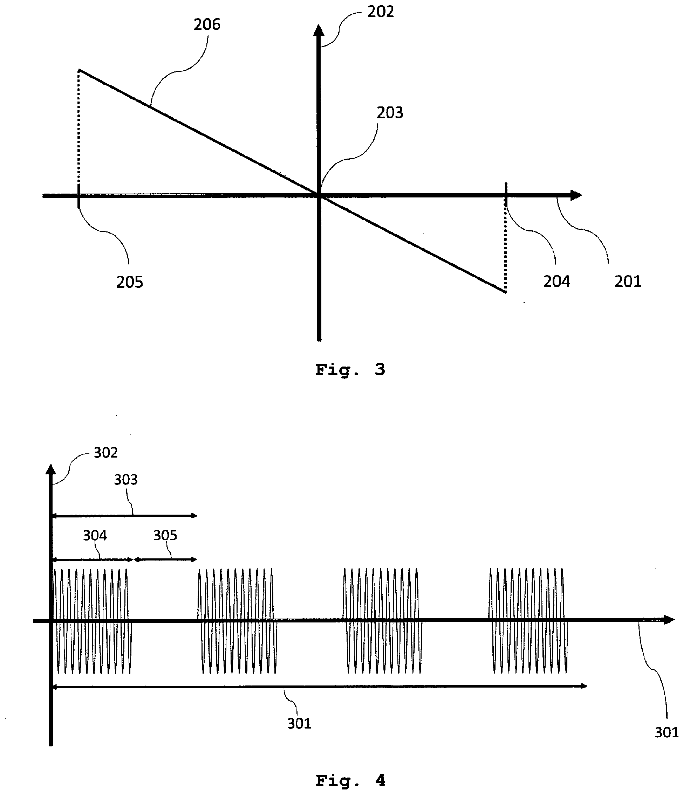

A substantial change in the pulses or environmental characteristics may be a change which is intended to significantly impact the ratio between expected and delivered values. For example, if the generator is able to deliver pulses in two frequency bands (e.g. around 900 kHz and around 2 MHz) and part of the circuit is specific to each frequency band, it may be decided that changing the frequency of the pulses is a substantial change in the pulse characteristics. Thus, if a user delivers a pulse k at 2 MHz after a series of pulses at 900 kHz, the history is reset and $\alpha_k$ = 1. A change in the pulse repetition frequency (PRF) (see Fig. 4) and/or a change in power may also be considered substantial changes in the pulse characteristics. Environmental conditions may, for example, designate the environmental conditions (e.g. a temperature) of the generator itself and/or of the load (e.g. the treatment head) .

In some embodiments, $\alpha_k$ may be reset to a value other than 1. For example, the reset value may be smaller than 1 to avoid overpower. In another example, a different value may be used for each generator, if it is detected in production that certain generators tend to be in underpower or overpower. Thus, a specific value can be used for each generator (e.g. stored in a configuration file). Starting value and reset value of the feedback control mechanism are preferably the same.

Defining the significance of the changes in pulse and/or environmental characteristics is preferably done *a priori* based on static criteria (e.g. established during the research and development process).

Additionally or alternatively, a significant change may be detected dynamically, e.g. by detecting that a value of a pulse are not consistent with the history in memory (e.g. $\eta_{k-1}$ may be different from the mean value of $\eta$ in history by more than twice the variance calculated over the last 10 values in history). In this case, it may not be possible to explicitly relate the change to a specific change in the pulse characteristics or environmental characteristics.

The pulse history may also be reset if the duration between the last pulse in the history and the current pulse is too long. This is advantageous since environmental conditions may change without being detected, and this avoids applying corrections which are not relevant anymore.

The feedback control mechanism may be adapted to select a portion of the pulse history. A portion of the pulse history may be a subset of the pulse history. The portion may be selected, for example, based on a similarity between current and historical pulse characteristics and/or environmental characteristics.

For example, the feedback control mechanism may comprise several histories depending on the pulse characteristics and/or environmental conditions or one history but selects the relevant pulses within the pulse history depending on the current pulse characteristics and/or environmental conditions.

In a preferred embodiment, several histories corresponding to (preferably predefined) pulse and/or environmental characteristics categories are kept in memory, and the feedback control mechanism considers the history which is relevant to the current pulse to be delivered.

This may be advantageous, for example, if a user delivers a series of pulses at a given frequency and subsequently switches to another frequency before coming back to the first frequency used. In this case, the history of the pulses at the first frequency may be used when the user comes back to the first frequency as they values were in the memory.

A pulse history may be formed by a table with, in rows, the different pulse frequencies available and, in columns, different power bins (e.g. corresponding to power intervals of 50 W). In each of the table's cells, the value $\eta$ of the last N pulse delivered with the appropriate characteristics are kept in the memory.

When continuous values are possible (e.g. for power), the width of the bins may preferably be defined such that:

- they are small enough so that two values within the same bin lead to substantially the same correction value, and
- large enough to avoid bins with no values.

Alternatively, the bins are not formally segregated, and all pulses are kept in memory. Then, the relevant pulses are selected dynamically for each pulse (e.g. all pulses delivered in the last 30 minutes at the same frequency, with the same PRF and with a requested power within 20 W of the currently requested power). Pulses' influence may also be weighted based on the proximity with current pulse's parameters (typically, the most recent pulses and/or the pulses with a closer power having more weight in the computation of the correction factor).

The ultrasonic transducer may be a piezoceramic transducer having a fundamental frequency f0 and a third harmonic 3f0. The first and second frequency bands of the radiofrequency generator may comprise f0 and 3f0, respectively.

As mentioned, 3f0 may substantially correspond to three times f0 with the usual uncertainty known by the skilled person.

In a preferred embodiment, the treatment head comprises the piezoceramic transducer, and the first and second frequency bands of the RF generator may comprise f0 and 3f0, respectively. For example, f0 may be at 1 MHz (preferably between 800 kHz and 1200 kHz, most preferably between 900 kHz and 1100 kHz), and 3f0 at substantially 3 MHz (preferably between 2400 kHz and 3600 kHz, most preferably between 2700 kHz and 3300 kHz).

A width of the first and/or second frequency bands may be at least 50 kHz, preferably at least 100 kHz.

This may be advantageous as it enables the RF generator to generate a signal at a frequency adapted to each treatment head. Preferably, the frequency bands are defined in the MCU.

Preferably, either one or both frequency bands is comprised within the interval [0.85 f, 1.15 f] around a frequency f.

Preferably, the treatment head comprises a memory and/or a treatment head identifier tag.

The memory may be shared with the HITU device, i.e. be the memory described herein above, or be a separate dedicated treatment head memory.

The memory may comprise a code portion which is suitable to identify the treatment head, e.g. a treatment head type such a serial number, as well as other characteristics of the treatment head.

The treatment head identifier tag may be any tag which is suitable to identify the treatment head, e.g. its type, for example an RFID tag, a barcode, a QR code, or similar.

A controller (e.g. a dedicated third controller or the first controller) may be adapted to read out a driving frequency saved on the memory of the treatment head, and/or read out a treatment head identifier value saved on the memory of the treatment head. A controller may obtain a treatment frequency based on the treatment head identifier value and/or obtain a treatment frequency based on the treatment head identifier tag.

It will be understood that the memory of the treatment head may comprise one or several driving frequencies, e.g. one driving frequency for each frequency band. It is conceivable that a first driving frequency is independent of the treatment head and thus always constant in one frequency band, but a second driving frequency varies by treatment head in the other frequency band, i.e. only the second frequency may need to be saved in the memory. It is also conceivable that there is a rule enabling to determine the value the second frequency band based on the value stored in the memory for a first frequency band.

In a preferred embodiment, preferred driving frequencies are stored for each treatment head, preferably in the memory of each treatment head, or in a remote database. Preferably, the HITU device also comprises a computer program wherein the computer program is able to read the preferred driving frequencies for the treatment head in use and, when the control of the user interface is activated, the first controller switches to the appropriate frequency band, and/or the output frequency of the RF generator is switched to a value within this frequency band corresponding to the value read in the memory of the treatment head, and/or the second controller changes the impedance matching for the impedance matching circuit corresponding to the output frequency band of the radiofrequency generator.

This may be advantageous because it enables each treatment head to be driven at optimal frequencies automatically, i.e. including less work for an operator and with lower error rates. Optimal frequencies may be chosen within certain frequency ranges, for example to maximize the efficacy (defined as the ratio between the emitted acoustic power and the output electrical power of the RF generator) or to minimize the amplitude of a prefocal peak, or as a compromise between several factors such as the efficacy and amplitude of a prefocal peak.

In a preferred embodiment, a list of preferred frequency values is stored in the treatment head or in the remote database corresponding to preferred driving frequency values for different focus depths under skin (or another value or set of values characterizing the location of the beam with respect to the patient's anatomy). These lists can also be functions (e.g. polynoms) enabling the definition of preferred driving frequency within a certain frequency band depending on the focus depth under skin (or another value or set of values characterizing the location of the beam with respect to the patient's anatomy). Preferably, there are at least two lists or functions, one for each frequency band. The invention is thus also directed to a treatment head comprising a memory on which at least one, preferably several, preferred driving frequencies are saved.

Additionally or alternatively, at least an intensity value characterizing the skin exposure (to heating and/or cavitation risk) for a given treatment head at a preferred frequency or within a certain frequency band is stored in the treatment head or in the remote database. Preferably, this intensity value is a function of the spatial peak instantaneous intensity (Isptp) at skin level (e.g. the maximum Isptp within all planes orthogonal to the main ultrasound propagation axis at which the skin can be located) at a predefined power. This enables to limit (preferably automatically, e.g., using a software) the maximum allowed power to avoid skin burns.

Preferably, the intensity value is a list of values corresponding to a frequency or a frequency band and to a list of possible depths under skin (or another value or set of values characterizing the location of the beam with respect to the patient's anatomy). This enables the adaptation of the maximum allowed power depending on each treatment head individual field characteristics (e.g. measured during a production process).

Preferably, intensity values or functions are stored for each frequency band (or preferred driving frequencies).

Intensity is actually a vector but is commonly approximated as a scalar using a plane wave approximation also in the field of HIFU, where the beams are strongly focused. Skin position with respect to the beam may also be characterized by a single value (e.g. the distance to the focus at the center of the beam, or minimum distance to focus within an imaging plane, or minimum distance to focus within the impinged skin area). Alternatively, more complex representations can be used to characterize skin exposure.

More generally, at least one of the following variables is preferably stored in the memory of the treatment head:

- Preferred driving frequency,
- Intensity value characterizing skin exposure;
- Therapy transducer efficacy (ratio characterizing the amount of electric power which is actually converted into acoustic power by the transducer).

These values may be single values, or list of values or functions and may depend on the following variables:

- Pulse type (which may be characterized by duration, frequency band, duty cycle, pulse repetition factor, focus speed, focus trajectory, size of the impinged zone, etc.),
- Location of the beam with respect to the patient's anatomy (e.g. focus depth under skin at the center of the beam),
- Acoustic power.

Thus, based on current settings (e.g. pulse type, location of the beam with respect to the patient's anatomy, acoustic power), a computer may be able to:

- set the RF generator to the appropriate frequency since based on the preferred driving frequency) ,and/or
- compute the maximum allowed power for the current treatment head since ibased on the intensity value characterizing skin exposure (which may, e.g. be compared it to a threshold, and/or
- compute an electric power needed by the RF generator to obtain a given acoustic power based on the efficacy of the transducer.

These values may be directly read in the memory of the treatment head or in the remote database. Alternatively, they may be determined based on values read. For example, an intensity value may be stored in the treatment head for each frequency band at an acoustic power of 100 W, and a linear estimation can be performed by a computer by multiplying this value by 2 if the acoustic power set by the user is set to 200 W. It may then be compared to a maximum value (e.g. stored in the memory of the computer) to determine if the intensity of the pulse may induce skin burns at 200 W. This is particularly advantageous to save time when characterizing the individual treatment heads, e.g. during a production process.

A controller (e.g. the first controller, the third controller, or a dedicated fourth controller) may also be adapted to read out an efficacy of the ultrasound transducer saved on the memory of the treatment head, and/or read out a treatment head identifier value saved on the memory of the treatment head, and/or obtain an efficacy of the ultrasound transducer based on the treatment head identifier value, and/or obtain an efficacy of the ultrasound transducer based on the treatment head identifier tag.

In a preferred embodiment, at least an efficacy value for the at least one transducer is stored, preferably in the memory of each treatment head or in a remote database. Preferably, the computer program is able to read the efficacy values for the treatment head in use such that, when a HITU pulse is delivered, the electric power requested to the radiofrequency generator is corrected using the appropriate stored efficacy value in order for the acoustic power at the output of the at least one HITU transducer to match the acoustic power requested by the user. These efficacy values can for example be determined in a production or maintenance process using acoustic balances as known in the art.

Thereby, when a HITU pulse is delivered, the electric power delivered to the radiofrequency generator may be corrected using the appropriate stored efficacy value in order for the acoustic power at the output of the at least one HITU transducer to match the acoustic power requested by the user.

In a preferred embodiment, the HITU device, preferably the RF generator, further comprises a memory (preferably in a MCU) storing at least one calibration curve for the radiofrequency generator, comprising at least a calibration curve enabling at least one of relating the driving signal (e.g. voltage or current or duty cycle) to the output power, translating output signal or a power measurement system to a value of emitted power and/or reflected power (e.g. the output voltage measured by a coupler to a value of emitted power and/or translating the output voltage measured by a coupler to a value of reflected power).

Preferably, the calibration curves are polynoms and are preferably determined for each individual radiofrequency generator and for each frequency band within each generator during a calibration. Preferably, they may be changed during a maintenance for example.

The HITU device may further comprise at least one calibration curve for the radiofrequency generator saved in the memory and/or in a dedicated device memory.

The calibration curve may enable at least one of:

- relating the driving voltage to the output power;
- translating the output voltage measured by a coupler to a value of emitted power;
- translating the output voltage measured by a coupler to a value of reflected power.

The calibration curves may be polynoms and are preferably defined for each individual RF generator and for each frequency band within each RF generator and/or for each PRF value

The RF generator may be adapted to provide a pulsed emission mode.

A pulsed emission mode may enable the definition and delivery of pulses made-up of periodic bursts having inter-bursts intervals during which the emission is stopped.

In a preferred embodiment, the RF generator comprises a switch able to periodically allow and interrupt the emission during a pulse according to a configuration (e.g. consisting in a pulse repetition frequency and a duty cycle).

The automatic gain control circuit and/or the software correction may be configured to maintain an output correction value of the AGC during an inter-bursts interval, preferably using a sample-and-hold circuit.

In a preferred embodiment, the generator also comprises a sample-and-hold circuit enabling to maintain the output correction value of the AGC during inter-bursts intervals. This is advantageous because, without the sample-and-hold circuit, when the signal emission is off during inter-bursts intervals, the AGC may cause an increase of the emitted RF signal since no output is measured. This signal would be without use since it is the desired behavior to periodically switch off the emission according to the PRF in this case. Since the AGC has a reaction time, this may translate into an artificially high correction when the emission resumes, shortly followed by a very low correction, translating into power oscillations. The sample-and-hold circuit may thus maintain the adjustment signal to a relevant value when the emission is off due to PRF.

Further disclosed is a method of treating a patient using a HITU device. The HITU device may be a HITU device as described herein above. The HITU device is adapted to deliver a pulse at at least two different pulse frequencies. The pulse frequency at which a pulse is delivered is selected based on a depth under the skin where the pulse is to be delivered and/or an occurrence of an hyperechoic mark of a previous pulse.

For example, the HITU device may be adapted to deliver pulses at a first pulse frequency, and a second pulse frequency which is higher than the first pulse frequency. For deeper depths under the skin, first pulse frequency may be selected leading to a deeper penetration at lower frequencies. At smaller depths under the skin, the second pulse frequency may be selected.

In some embodiments, a limited number of pulses may be validated (in terms of safety and/of effectiveness). For example, the HITU device may be adapted to deliver two types of pulses: a first pulse may be at a first lower frequency and may have a duration of 1 s, and the second pulse at a higher frequency which may have a duration of 0.5 s. Thus, the frequency may not be the only parameter which changes between available pulse types. In this example, it may happen that a patient does not tolerate the pain induced by the 1 s pulse. The user may decide to switch to the 0.5 s pulse to manage pain, despite the 1 s pulse being still efficient at the current depth. In this example, switching frequency is not performed due to a variation of the depth under skin of the target.

In the following, the invention is described in detail with reference to the following figures, showing:

Fig. 1: a schematic drawing of a preferred embodiment.

Fig. 2: a schematic illustration of different implementations of a system.

Fig. 3: a schematic depiction of a correction factor.

Fig. 4: a schematic representation of the voltage output.

Fig. 5a-5b: a schematic representation of a preferred embodiment enabling to switch between two frequency bands.

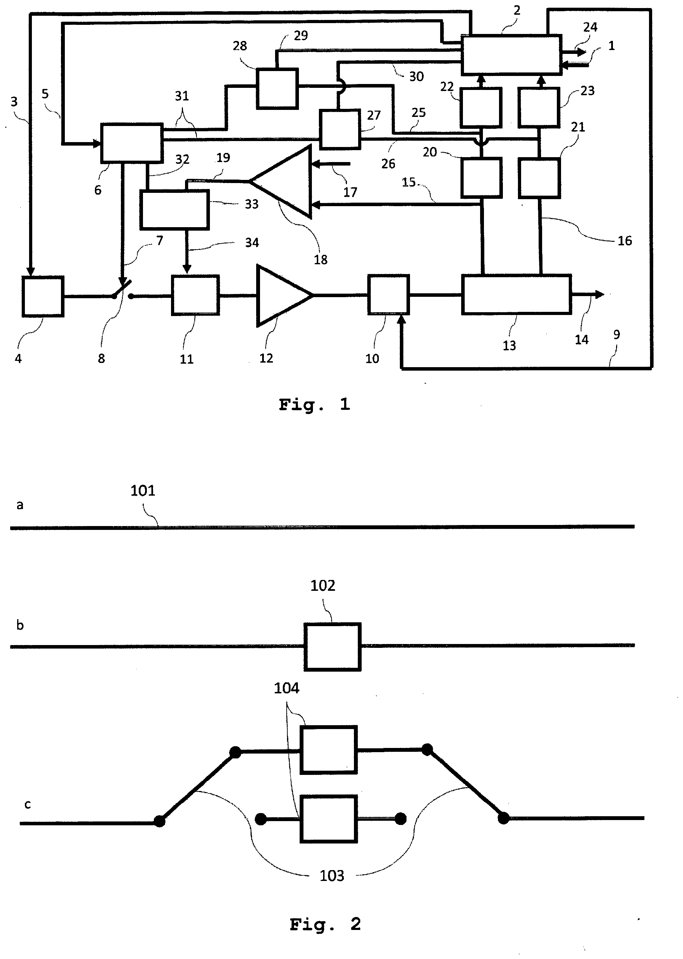

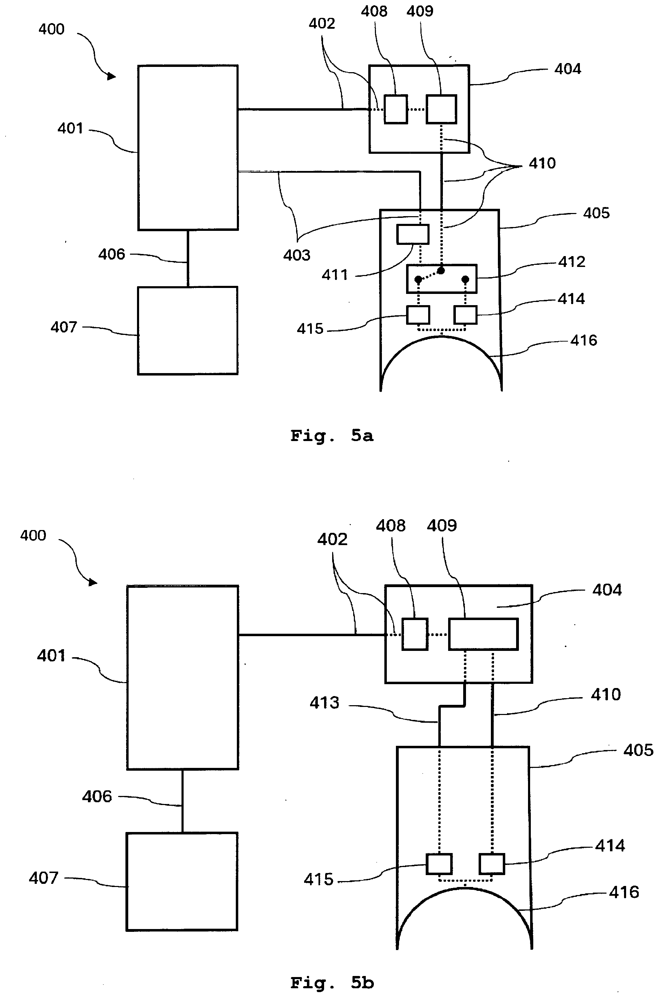

Fig. 1 shows a schematic drawing of a preferred concept of a HITU device (see Figs. 5a-5b). Via a software (e.g. a user interface providing a input device for a user), a command 1 including a frequency, a power, a duration, a pulse repetition frequency (PRF), a starting time, and/or one or several protection thresholds (e.g. an overpower threshold and/or a reflected power threshold) is sent to a MCU 2. The MCU sends a configuration command 3 to a signal generator 4 (e.g. a sine generator). To start the power emission, the MCU 2 sends an emission control signal 5 to a Complex Programmable Logic Device (CPLD) 6, which then send a command 7 to a switch 8 enabling the pulse emission and controlling the PRF. For example, the function of the switch 8 can practically be implemented using two switches: one for enabling the pulse emission, and a second one responsible for the control of the PRF. The output power is set by changing a gain or attenuation value 11. The signal is then amplified (e.g. using one or several fixed amplifiers 12) and filtered by an appropriate filter 10, which results in an output signal 14. The filter 10 can be made-up of one or several filters and is configured (e.g. one filter among two is selected depending on the desired output frequency) by a signal 9 sent by the MCU 2. A coupler 13 sends an attenuated signal 15 corresponding to the forward power 14 to an automated gain control circuit (AGC) 18. In the AGC 18, the measured forward power 15 is compared to a power setpoint 17, which results in a signal 19. In order for the AGC 18 to send a proper signal 34 adapting the value of the gain or attenuation value 11, a sample-and-hold circuit 33 is used. This sample-and-hold circuit is configured to the proper PRF (e.g. by a signal 32 from the CPLD 6). Without this sample-and-hold circuit 33, when the signal emission is off due to PRF, the AGC 18 may otherwise ask for an increase of the emitted RF signal because the signal 15 is absent. This signal may be irrelevant since it is the desired behavior to periodically switch off the emission according to the PRF. Since the AGC 18 has a reaction time, this would translate into an artificially high correction when the emission resumes, thus to unwanted power oscillations. The sample-and-hold circuit 33 thus has the role of maintaining the adjustment signal 34 to a relevant value when the emission is off due to PRF. Preferably, the coupler 13 is a bidirectional coupler, and also sends an attenuated signal 16 corresponding to the reverse power. Preferably, RF detectors 20 and 21 convert the voltages 15 and 16 resulting from the bi-directional coupler 13 into power values. These converted signals are then preferably averaged by averagers 22 and 23 and converted by analog to digital converters (ADC), preferably in the MCU 2. Preferably, the MCU 2 applies a correction (e.g. a linear correction depending on the frequency) compensating for the fact that the RF converter was calibrated at certain frequencies (e.g. 1 and 3 MHz), but potentially used at a slightly different frequency (e.g. 1.02 MHz). Preferably at least one corrected value 24 (e.g. the forward power and/or the reverse power) is communicated to a software, preferably a software comprising a user interface, e.g. for display or logging purposes.

Preferably at least one corrected value (e.g. the forward power 25 and/or the reverse power 26) is sent to a comparator (27 for reverse power and/or 28 for forward power) and compared to at least a threshold 30 related to reverse power (e.g. a maximum reverse power value) and/or 29 related to forward power (a maximum output power value, e.g. 120% of the power setpoint, and/or a minimum output power value, e.g. 80% of the power setpoint). Then, preferably, if at least one of the values is beyond the threshold, the emission is interrupted, e.g. by sending a command signal 31 to the CPLD 6. For example, the amplification 12 can be practically realized by several amplification stages. The filter 10 could also be in the treatment head for example, but it is advantageous to have it within the RF generator to filter undesired frequencies as early as possible, for example to avoid the need to dissipate energy in proximity to the patient. Filtering may also be omitted if all other stages (e.g. the amplification stages) do not generate significant unwanted frequencies.

Typical embodiments have a signal generator 4, a switch 8, and at least an amplification stage 12. Preferred embodiments comprise a logical unit (MCU, CPLD, etc.) able to command the generation of a signal at at least two different frequencies and/or within at least two frequency bands; a signal generator and optionally amplification stages and/or a variable gain or attenuation to generate the signal with the desired power; a switch to trigger the emission of the signal.

Fig. 2 shows a schematic drawing illustrating different filtering options represented in Fig. 1 (see filter 10) implemented differently depending on the specificities of the system.

For example and shown in panel (a), if filter (see Fig. 1) is omitted, the filtering stage is schematically represented by a line 101 (i.e. absence of dedicated filter, replacing filter 10 in Fig. 1), e.g. if the amplified signal does not comprise a significant part of the energy in undesired frequencies.

Alternatively, as shown in panel (b), a filter may be implemented using a single band-pass filter 102, which encompasses the two frequency bands.

Alternatively and as shown in panel (c), a filter may also be implemented as a combination of two switches 103 and two filters 104, each filtering the signal in of two frequency bands.

An impedance matching (which is preferably placed in the treatment head) may be omitted, e.g. if the ultrasound transducer impedance is naturally close to 50 Ohms. Alternatively, similar to Fig. 2b, a single broadband impedance matching encompassing the two frequency bands may also be used.

Alternatively, for example and similar to Fig. 2c, it may also be implemented as a combination of two switches 103 and two impedance matching, each of the two being responsible for the impedance matching in a different frequency band.

Fig. 3 depicts a correction factor which depends on the frequency. Depending on the output frequency 201, a correction factor 202 is defined, which translates into a correction function 206 (in the shown example as a linear correction function). The correction may be zero at a calibration frequency 203 and is computed within a frequency range between a minimum frequency 205 and a maximum frequency 204 preferably centered on the calibration frequency 203. The correction accounts for frequency-dependent variations when using the generator at frequencies slightly different from the calibration frequency. Preferably, this correction is applied by a MCU(see Fig. 1). Preferably, there are two different corrections, one around f0 (e.g. 1 MHz) and one around 3f0 (e.g. 3 MHz). Such corrections enable to show and/or log correct power values to the user and/or to properly implement safety thresholds (e.g. a maximum overpower threshold, or a maximum reverse power threshold).

Fig. 4: schematic representation of the voltage output 302 of a RF generator along time 301 during a HITU pulse in "pulsed" mode. Emission is not on during the whole duration 301 of the pulse. Emission is successively switched on during bursts 304 and off during inter-burst intervals 305. The Pulse Repetition Frequency (PRF) does not refer to the repetition frequency of the pulses but it refers to the repetition frequency of the bursts within the pulses, which may be an inverse of the duration of a burst and a subsequent interval. Typically, the pulsed mode is also described by a duty cycle defined as the product of the burst duration 303 by the PRF.

Fig. 5a shows a schematic drawing of a preferred concept of a HITU device 400 enabling to switch between two frequency bands. A first controller 401 sends a first configuration signal 402 to a second controller 408 within a RF generator 404, and a second configuration signal 403 to a third controller 411 within a treatment head 405, based on a signal 406 coming from a user interface 407. It will be understood that instead of a user interface 407 an automatic procedure to change frequency bands may be used, e.g. based on an ultrasound scanner which generated images which are analyzed by an image processing algorithm and which triggers the automatic decision of changing the frequency band. The controller 408 configures the RF power generation part 409 of the RF generator for the desired frequency band. A treatment head controller 411 is arragned in a treatment head 405. The treatment head controller 411 activates a switch 412 to direct the output 410 of the RF generator 404 to the appropriate impedance matching circuit 414 or 415, which will transmit it to a transducer 416 where it will be converted to ultrasound beam.

Fig. 5b shows an embodiment which is similar to the one shown in Fig. 5a. Here, no switch or controller is arranged within treatment head 405. It will be understood that identical features with the same reference numerals are not described again, for clarity. The RF generator 404 sends a signal 413 or 410, via controller 408 and RF power generation part 409, at the appropriate frequency to the treatment head 405. If the signal is a signal 413 within the first frequency band, it reaches the transducer 416 through the impedance matching 415. If the signal is a signal 410 within the second frequency band, it reaches the transducer 416 through the impedance matching 414.

**Claims**

1. A HITU device (400) comprising a radiofrequency generator (404), a first controller (408), and a user interface (407), wherein

the radiofrequency generator (404) is configured for delivering a, preferably high-power, radiofrequency signal (410) within at least a first frequency band and a second frequency band, and wherein

the first controller (408) is configured for switching between the first and the second frequency band, and wherein either

the user interface (407) comprises a switch which, when activated, causes the first controller (408) to change the output frequency of the radiofrequency generator (404) from the first to the second frequency band or from the second to the first frequency band, and/or the first controller is adapted to automatically switch the output frequency of the radiofrequency generator (404) from the first to the second frequency band or from the second to the first frequency band, and preferably to display an indication that a change occurred on the user interface (407).

2. The HITU device (400) according to claim 1, further comprising a treatment head (405) and wherein the treatment head (405) comprises an ultrasonic transducer (416).

3. The HITU device (400) according to any one of the preceding claims, comprising

at least a first impedance matching circuit (414) and a second impedance matching circuit (415), preferably wherein the first impedance matching circuit (414) and second impedance matching circuit (415) are assigned to the first and the second frequency bands, respectively, optionally a second controller (411) configured to switch between the first impedance matching circuit (414) and the second impedance matching circuit (415), and further wherein the switch of the user interface (407), when activated, causes the second controller (411) to switch from the first impedance matching circuit (414) to the second impedance matching circuit (415) or from the second impedance matching circuit (415) to the first impedance matching circuit (414), preferably in synchronization with the first controller such that the impedance matching circuit (414, 415) which is assigned to a currently selected output frequency is selected.

4. The HITU device (400) according to any one of the preceding claims, further comprising a frequency filter (10) each for the first frequency bands and/or second frequency bands, wherein the frequency filter (10) is configured to filter out at least the second and/or third harmonics of the corresponding frequency band.

5. The HITU device (400) according to any one of the preceding claims, wherein the user interface (407) is adapted to display a medical image, preferably an ultrasound image, with a representation of at least one of

- an affected region of a tissue,
- safety zones,
- characteristic points of the HITU field based on a selected bandwidth or the selected frequency band.

6. The HITU device (400) according to any one of the preceding claims wherein an output power of the RF generator (404) is above 200 W, preferably above 500 W.

7. The HITU device (400) according to any one of the preceding claims wherein the RF generator (404) further comprises an automatic gain control circuit and/or a software correction, such that an output power is within 20%, preferably within 5%, of a setpoint.

8. The HITU device (400) according to any one of the preceding claims, further having a feedback control mechanism configured to adapt a pulse characteristic based on a pulse history.

9. The HITU device (400) according to claim 8, wherein the feedback control mechanism is configured to reset the pulse history if a change in pulse characteristics or environmental characteristics is above a threshold.

10. The HITU device (400) according to any one of claims 8 or 9, wherein the feedback control mechanism is adapted to select a portion of the pulse history, preferably based on a similarity between current and historical pulse characteristics and/or environmental characteristics.

11. The HITU device (400) according to any one of claims 2 to 10, wherein the ultrasonic transducer (416) is a piezoceramic transducer having a fundamental frequency f0 and a third harmonic 3f0, and wherein the first and second frequency bands of the RF generator (404) comprise f0 and 3f0, respectively.

12. The HITU device (400) according to any one of the preceding claims, wherein a width of the first and/or second frequency bands is at least 50 kHz, preferably at least 100 kHz.

13. The HITU device (400) according to any one of the claims 2 to 12, wherein the treatment head (405) comprises a memory and/or a treatment head identifier tag.

14. The HITU device (400) according to claim 13, wherein a third controller is adapted to

   - read out a driving frequency saved on the memory of the treatment head (405), and/or
   - read out a treatment head identifier value saved on the memory of the treatment head (405), and obtain a treatment frequency based on the treatment head identifier value, and/or
   - obtain a treatment frequency based on the treatment head identifier tag.

15. The HITU device (400) according to claim 13, wherein a fourth controller is adapted to:

   - read out an efficacy of the ultrasound transducer (416) saved on the memory of the treatment head (405), and/or
   - read out a treatment head identifier value saved on the memory of the treatment head (405), and obtain an efficacy of the ultrasound transducer (416) based on the treatment head identifier value, and/or
   - obtain an efficacy of the ultrasound transducer (416) based on the treatment head identifier tag.

16. The HITU device (400) according to any one of the preceding claims, further comprising a device memory for storing at least one calibration curve for the radiofrequency generator.

17. The HITU device (400) according to any of the preceding claims, wherein the RF generator (404) is adapted to provide a pulsed emission mode.

18. The HITU device (400) according to claim 7 and 17, wherein the automatic gain control circuit and/or the software correction is configured to maintain an output correction value of the AGC during an inter-bursts intervals, preferably using a sample-and-hold circuit.

19. A method of treating a patient using a HITU device (400), preferably a HITU device (400) according to any one of the preceding claims, wherein the HITU device is adapted to deliver a pulse at at least two different pulse frequencies, and wherein the pulse frequency is selected based on at least one of a depth under the skin where the pulse is to be delivered and/or an occurrence of an hyperechoic mark of a previous pulse.

Fig. 1

Fig. 2

Fig. 3

Fig. 4

Fig. 5a

Fig. 5b

Europäisches
Patentamt

European
Patent Office

Office européen
des brevets

# EUROPEAN SEARCH REPORT

**Application Number**

EP 25 31 5017

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/233886 A1 (SHOUDY DAVID ANDREW [US] ET AL) 28 July 2022 (2022-07-28) | 1,2,9, 10,12, 16-19 | INV.<br>A61N7/02<br>A61B8/00 |
| Y | * paragraphs [0034], [0035], [0041], [0042], [0048] *<br>* figure 1 * | 3-8,11, 13-15 | ADD.<br>A61B90/00<br>A61N7/00 |
| X | US 2017/360413 A1 (ROTHBERG JONATHAN M [US] ET AL) 21 December 2017 (2017-12-21)<br>* paragraphs [0113], [0116] *<br>* figures 1A,1B * | 1,2 | |
| X | US 2013/226000 A1 (KIM BAE HYUNG [KR] ET AL) 29 August 2013 (2013-08-29)<br>* paragraphs [0012], [0023], [0049], [0050] *<br>* figure 1 * | 1,2 | |
| Y | US 6 236 274 B1 (LIU SHIH-PING [TW]) 22 May 2001 (2001-05-22)<br>* claim 1 * | 3,4 | TECHNICAL FIELDS SEARCHED (IPC) |
| Y | US 2010/280373 A1 (FAN LIEXIANG [US] ET AL) 4 November 2010 (2010-11-04)<br>* paragraph [0075] *<br>* figure 3 * | 5 | A61N<br>A61B |
| Y | US 2015/105701 A1 (MAYER CARL [US] ET AL) 16 April 2015 (2015-04-16)<br>* paragraph [0098] * | 6 | |
| Y | US 2024/293132 A1 (DECASPER MICHAEL THOMAS [US] ET AL) 5 September 2024 (2024-09-05)<br>* paragraph [0045] * | 7,8 | |
| Y | US 2015/005634 A1 (CURRA FRANCESCO P [US] ET AL) 1 January 2015 (2015-01-01)<br>* paragraphs [0032], [0033] * | 11 | |

-/--

The present search report has been drawn up for all claims

| Place of search | Date of completion of the search | Examiner |
|---|---|---|
| The Hague | 1 July 2025 | Milles, Julien |

EPO FORM 1503 03.82 (P04C01)

page 1 of 2

Europäisches
Patentamt

European
Patent Office

Office européen
des brevets

## EUROPEAN SEARCH REPORT

Application Number

EP 25 31 5017

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2021/275834 A1 (DUCHON DOUGLAS J [US] ET AL) 9 September 2021 (2021-09-09) * paragraph [0080] * ----- | 13-15 | |
| | | | **TECHNICAL FIELDS SEARCHED (IPC)** |

The present search report has been drawn up for all claims

| Place of search | Date of completion of the search | Examiner |
|---|---|---|
| The Hague | 1 July 2025 | Milles, Julien |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.................................................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 31 5017

This annex lists the patent family members relating to the patent documents cited in the above-mentioned European search report.
The members are as contained in the European Patent Office EDP file on
The European Patent Office is in no way liable for these particulars which are merely given for the purpose of information.

01-07-2025

| Patent document cited in search report | | Publication date | Patent family member(s) | | Publication date |
|---|---|---|---|---|---|
| US 2022233886 | A1 | 28-07-2022 | CN | 117693382 A | 12-03-2024 |
| | | | EP | 4284503 A1 | 06-12-2023 |
| | | | JP | 7592882 B2 | 02-12-2024 |
| | | | JP | 2024505502 A | 06-02-2024 |
| | | | JP | 2025028068 A | 28-02-2025 |
| | | | US | 2022233886 A1 | 28-07-2022 |
| | | | US | 2025090874 A1 | 20-03-2025 |
| | | | WO | 2022164701 A1 | 04-08-2022 |
| US 2017360413 | A1 | 21-12-2017 | AU | 2017281012 A1 | 06-12-2018 |
| | | | AU | 2022218473 A1 | 08-09-2022 |
| | | | CA | 3026277 A1 | 28-12-2017 |
| | | | CN | 109310395 A | 05-02-2019 |
| | | | CN | 114652344 A | 24-06-2022 |
| | | | EP | 3471622 A1 | 24-04-2019 |
| | | | EP | 4218594 A2 | 02-08-2023 |
| | | | JP | 7101126 B2 | 14-07-2022 |
| | | | JP | 2019523685 A | 29-08-2019 |
| | | | JP | 2022141730 A | 29-09-2022 |
| | | | KR | 20190020101 A | 27-02-2019 |
| | | | TW | 201801679 A | 16-01-2018 |
| | | | TW | 201919543 A | 01-06-2019 |
| | | | US | 2017360397 A1 | 21-12-2017 |
| | | | US | 2017360413 A1 | 21-12-2017 |
| | | | US | 2023089630 A1 | 23-03-2023 |
| | | | WO | 2017222964 A1 | 28-12-2017 |
| US 2013226000 | A1 | 29-08-2013 | KR | 20130080087 A | 12-07-2013 |
| | | | US | 2013226000 A1 | 29-08-2013 |
| US 6236274 | B1 | 22-05-2001 | TW | 445704 B | 11-07-2001 |
| | | | US | 6236274 B1 | 22-05-2001 |
| US 2010280373 | A1 | 04-11-2010 | CN | 101879077 A | 10-11-2010 |
| | | | DE | 102010018857 A1 | 16-12-2010 |
| | | | JP | 5661329 B2 | 28-01-2015 |
| | | | JP | 2010259806 A | 18-11-2010 |
| | | | KR | 20100120091 A | 12-11-2010 |
| | | | KR | 20160056867 A | 20-05-2016 |
| | | | US | 2010280373 A1 | 04-11-2010 |
| US 2015105701 | A1 | 16-04-2015 | NONE | | |
| US 2024293132 | A1 | 05-09-2024 | US | 2024293132 A1 | 05-09-2024 |
| | | | WO | 2022182648 A1 | 01-09-2022 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 25 31 5017

This annex lists the patent family members relating to the patent documents cited in the above-mentioned European search report.
The members are as contained in the European Patent Office EDP file on
The European Patent Office is in no way liable for these particulars which are merely given for the purpose of information.

01-07-2025

| Patent document cited in search report | | Publication date | Patent family member(s) | | Publication date |
|---|---|---|---|---|---|
| US 2015005634 | A1 | 01-01-2015 | US | 2012232388 A1 | 13-09-2012 |
| | | | US | 2015005634 A1 | 01-01-2015 |
| US 2021275834 | A1 | 09-09-2021 | AU | 2017302612 A1 | 14-03-2019 |
| | | | AU | 2022235565 A1 | 13-10-2022 |
| | | | AU | 2024227630 A1 | 14-11-2024 |
| | | | CA | 3032390 A1 | 01-02-2018 |
| | | | DK | 3490673 T3 | 07-04-2025 |
| | | | EP | 3490673 A1 | 05-06-2019 |
| | | | EP | 4520340 A2 | 12-03-2025 |
| | | | US | 2021275834 A1 | 09-09-2021 |
| | | | WO | 2018022968 A1 | 01-02-2018 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0232506 A1
- US 20060206105 A1
- US 20180036558 A1
- US 9931245 B2
- US 5558092 A